# EUROPEAN PATENT APPLICATION

(11) **EP 4 269 993 A1**
(43) Date of publication of application: **01.11.2023**
(21) Application number: 21915132.1
(22) Date of filing: 20.12.2021
(51) Int. Cl.: G01N 27/00, G01N 27/06, G01N 27/22

(54) **FLUID STATE DETECTION SENSOR**

(30) Priority: 28.12.2020 JP 2020218839
(71) Applicant: KYB Corporation, Minato-ku, Tokyo 105-5128 (JP)
(72) Inventor: NAKAMURA, Ryohei, Tokyo 105-5128 (JP); KAMEDA, Yukinori, Tokyo 105-5128 (JP); NAGAI, Yuki, Tokyo 105-5128 (JP); YOSHIDA, Takahiro, Tokyo 105-5128 (JP)
(74) Representative: Grünecker Patent- und Rechtsanwälte PartG mbB
(86) International application number: PCT/JP2021/047016
(87) International publication number: WO 2022/145272

(57) **Abstract**

A fluid state detection sensor (1) includes: an electrical conductivity calculation unit (30) having an electrical conductivity calculation part (32) configured to calculate an electrical conductivity of a fluid from a voltage value acquired in a first voltage acquisition part (31); and a relative dielectric constant calculation unit (40) having a relative dielectric constant calculation part (42) configured to calculate a relative dielectric constant of the fluid from a voltage value acquired in a second voltage acquisition part (41), wherein the first voltage acquisition part (31) acquires an inter-electrode voltage value after a predetermined time period after a switching part (20) above a connection target of a pair of electrodes (11, 12) to the electrical conductivity calculation unit (30), and the second voltage acquisition part (41) acquires the inter-electrode voltage value after the predetermined time period after the switching part (20) switched the connection target of the pair of electrodes (11, 12) to the relative dielectric constant calculation unit (40).

## Description

### TECHNICAL FIELD

The present invention relates to a fluid state detection sensor.

### BACKGROUND ART

In a device provided with a hydraulic actuator, such as a hydraulic cylinder, an apparatus is driven with a working oil that is supplied from a hydraulic pump. In the apparatus, in which the working oil is supplied so as to be circulated therein, in order to allow a smooth operation of the apparatus in a continuous manner, a fluid state detection sensor for detecting a degradation state of the working oil is provided. In the fluid state detection sensor of this type, a pair of electrodes are arranged in a flow passage of the working oil by being provided so as to be opposed with each other in parallel. By applying voltage to the pair of electrodes and by measuring an inter-electrode voltage value, a relative dielectric constant and an electrical conductivity of the working oil are calculated, and a state of the working oil is detected on the basis of thus-calculated relative dielectric constant and electrical conductivity of the working oil (for example, see JP2017-198528A).

### SUMMARY OF INVENTION

In the fluid state detection sensor as described above, the inter-electrode voltage value is acquired by switching a connection target of the electrodes between an electrical conductivity measurement circuit and a relative dielectric constant measurement circuit. However, when the connection target of the electrodes is switched to the electrical conductivity measurement circuit or the relative dielectric constant measurement circuit, a surge voltage is accumulated in an inter-electrode voltage due to factors such as an accumulation state of electric charge in the electrodes, a degradation state of the working oil, and so forth, and there is a risk in that the inter-electrode voltage becomes unstable. In addition, because the electrical conductivity and the relative dielectric constant are calculated on the basis of the inter-electrode voltage value acquired from the inter-electrode voltage in the unstable state, there is a problem in that measurement accuracies for the electrical conductivity and the relative dielectric constant are deteriorated.

An object of the present invention is to provide a fluid state detection sensor that suppresses deterioration of measurement accuracies for an electrical conductivity and a relative dielectric constant and that accurately detects a state of a fluid.

According to one aspect of the present invention, a fluid state detection sensor configured to detect a state of a fluid includes: a pair of electrodes arranged in a flow passage of the fluid, the pair of electrodes being provided so as to be opposed to each other; an electrical conductivity calculation unit having a first voltage acquisition part configured to acquire an inter-electrode voltage value via the pair of electrodes and an electrical conductivity calculation part configured to calculate an electrical conductivity of the fluid from the voltage value acquired in the first voltage acquisition part; a relative dielectric constant calculation unit having a second voltage acquisition part configured to acquire an inter-electrode voltage value via the pair of electrodes and a relative dielectric constant calculation part configured to calculate a relative dielectric constant of the fluid from the voltage value acquired in the second voltage acquisition part; and a switching part configured to switch a connection target of the pair of electrodes to the electrical conductivity calculation unit or the relative dielectric constant calculation unit, the first voltage acquisition part acquires the inter-electrode voltage value after a predetermined time period after the switching part switched the connection target of the pair of electrodes to the electrical conductivity calculation unit, and the second voltage acquisition part acquires the inter-electrode voltage value after a predetermined time period after the switching part switched the connection target of the pair of electrodes to the relative dielectric constant calculation unit.

### BRIEF DESCRIPTION OF DRAWINGS

[FIG. 1] FIG. 1 is a diagram showing an electrical configuration of a fluid state detection sensor according to the present embodiment.
[FIG. 2] FIG. 2 is a diagram showing an electrical conductivity calculation processing flow performed by the fluid state detection sensor according to the present embodiment.
[FIG. 3] FIG. 3 is a diagram showing an inter-electrode voltage value acquisition timing for the fluid state detection sensor according to the present embodiment.
[FIG. 4] FIG. 4 is a diagram showing a relative dielectric constant calculation processing flow performed by the fluid state detection sensor according to the present embodiment.

### DESCRIPTION OF EMBODIMENTS

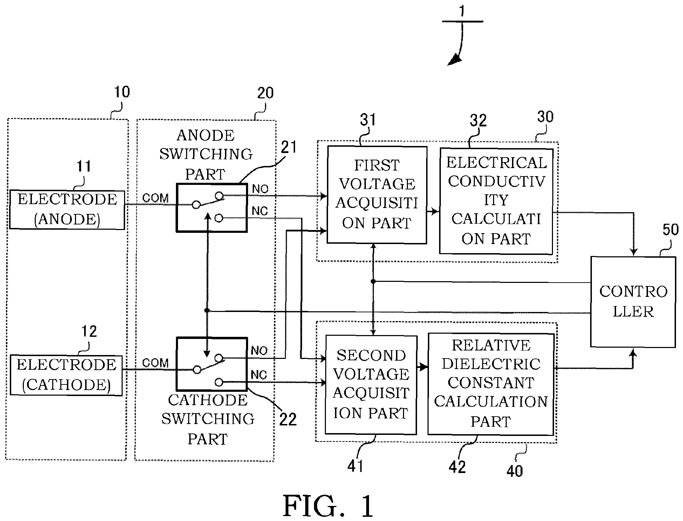

In the following, a fluid state detection sensor 1 according to this embodiment will be described with reference to FIGs. 1 to 4. Constituent elements in the following embodiment can be appropriately replaced with conventional constituent elements, etc., and various variations including combinations with other conventional constituent elements are possible. Therefore, the content of the present invention described in the Claims is not limited to the following description of the embodiment.

### < Electrical Configuration of Fluid State Detection Sensor 1>

The fluid state detection sensor 1 according to this embodiment is a sensor that detects, for example, a state of oil (fluid) flowing through a flow passage, and as shown in FIG. 1, the fluid state detection sensor 1 is provided with an electrode part 10, an electrical conductivity calculation unit 30 that calculates an electrical conductivity on the basis of an inter-electrode voltage value of the electrode part 10, a relative dielectric constant calculation unit 40 that calculates a relative dielectric constant on the basis of the inter-electrode voltage value of the electrode part 10, a switching part 20 that switches a connection target of the electrode part 10 to the electrical conductivity calculation unit 30 or the relative dielectric constant calculation unit 40, and a controller 50 that controls the switching part 20. The electrical conductivity calculation unit 30, the relative dielectric constant calculation unit 40, and the controller 50 form a function of a CPU.

The electrode part 10 has, as a pair of electrodes, an anode 11 and a cathode 12. The anode 11 and the cathode 12 are flat plates having the same shape, and they are provided so as to oppose to each other. The electrode part 10 is provided in an oil flow passage such that the oil flows between the anode 11 and the cathode 12. The anode 11 and the cathode 12 are not limited to the flat plate electrodes, and they may be cylindrical electrodes, comb-shaped electrodes, and so forth.

The electrical conductivity calculation unit 30 has a first voltage acquisition part 31 that is capable of acquiring the inter-electrode voltage value between the anode 11 and the cathode 12 of the electrode part 10 and an electrical conductivity calculation part 32 that calculates the electrical conductivity of the oil between the anode 11 and the cathode 12 on the basis of the inter-electrode voltage value acquired by the first voltage acquisition part 31. The first voltage acquisition part 31 is connected to a NO (normally open) terminal of an anode switching part 21, which is connected to the anode 11, and to the NO terminal of a cathode switching part 22, which is connected to the cathode 12. In other words, the first voltage acquisition part 31 is selectively connected to the anode 11 and the cathode 12 of the electrode part 10 and acquires the inter-electrode voltage value of the electrode part 10. The first voltage acquisition part 31 is connected to the electrical conductivity calculation part 32 and the controller 50 and outputs the acquired inter-electrode voltage value of the electrode part 10 to the electrical conductivity calculation part 32. The electrical conductivity calculation part 32 obtains a resistance component between the anode 11 and the cathode 12 of the electrode part 10 on the basis of the voltage value output from the first voltage acquisition part 31 and obtains the electrical conductivity of a liquid on the basis of the obtained resistance component.

The relative dielectric constant calculation unit 40 has a second voltage acquisition part 41 that is capable of acquiring the inter-electrode voltage value between the anode 11 and the cathode 12 of the electrode part 10 and a relative dielectric constant calculation part 42 that calculates the relative dielectric constant of the oil between the anode 11 and the cathode 12 on the basis of the inter-electrode voltage value acquired by the second voltage acquisition part 41. The second voltage acquisition part 41 is connected to a NC (normally close) terminal of the anode switching part 21, which is connected to the anode 11, and to the NC terminal of the cathode switching part 22, which is connected to the cathode 12. In other words, the second voltage acquisition part 41 is selectively connected to the anode 11 and the cathode 12 of the electrode part 10 and acquires the inter-electrode voltage value of the electrode part 10. The second voltage acquisition part 41 is connected to the relative dielectric constant calculation part 42 and the controller 50 and outputs the acquired inter-electrode voltage value of the electrode part 10 to the relative dielectric constant calculation part 42. The relative dielectric constant calculation part 42 obtains a resistance component between the anode 11 and the cathode 12 of the electrode part 10 on the basis of the voltage value output from the second voltage acquisition part 41 and obtains the relative dielectric constant of the liquid on the basis of the obtained resistance component.

Note that, in this embodiment, the electrical conductivity calculation unit 30 and the relative dielectric constant calculation unit 40 respectively have the voltage acquisition parts for acquiring the inter-electrode voltage value (the first voltage acquisition part 31 and the second voltage acquisition part 41). The configuration is not limited thereto, and it may be possible to employ a configuration in which the electrical conductivity calculation unit 30 and the relative dielectric constant calculation unit 40 have a single voltage acquisition part in common.

The switching part 20 has the anode switching part 21 that switches the connection target of the anode 11 and the cathode switching part 22 that switches the connection target of the cathode 12. The anode switching part 21 and the cathode switching part 22 are each formed by using a changeover-contact relay and respectively have a COM terminal, the NO terminal, and the NC terminal. In the anode switching part 21, the COM terminal is connected to the anode 11, the NO terminal is connected to the electrical conductivity calculation unit 30, and the NC terminal is connected to the relative dielectric constant calculation unit 40.

In the cathode switching part 22, the COM terminal is connected to the cathode 12, the NO terminal is connected to the electrical conductivity calculation unit 30, and the NC terminal is connected to the relative dielectric constant calculation unit 40. The anode switching part 21 and the cathode switching part 22 are connected to the controller 50. With a switching signal (an ON/OFF signal) from the controller 50, serving as an output signal, the connection target of the electrode part 10 is switched to either of the electrical conductivity calculation unit 30 or the relative dielectric constant calculation unit 40.

Note that, in order to measure the electric property of the oil that is a non-conducting material, it is required to capture an extremely minute electrical signal, and so, if the measurement of the electric property is performed in a state in which a unit, which is not required for the measurement, is connected, a measurement error is caused due to, for example, an increase in a leak current or a stray capacitance. Therefore, in the fluid state detection sensor 1, as described above, the electrical conductivity calculation unit 30 and the relative dielectric constant calculation unit 40 are completely separated by the switching part 20.

The controller 50 controls the switching part 20 to switch the connection target of the electrode part 10. In the fluid state detection sensor 1, when the electrical conductivity is to be calculated, the controller 50 controls the switching part 20 with the switching signal to switch the connection target of the electrode part 10 to the electrical conductivity calculation unit 30. Next, the controller 50 outputs a voltage acquisition start signal to instruct the first voltage acquisition part 31 to acquire the inter-electrode voltage value of the electrode part 10. The first voltage acquisition part 31 outputs the acquired inter-electrode voltage value to the electrical conductivity calculation part 32. The electrical conductivity calculation part 32 calculates the electrical conductivity on the basis of the output inter-electrode voltage value and outputs the electrical conductivity to the controller 50.

In addition, in the fluid state detection sensor 1, when the relative dielectric constant is to be calculated, the controller 50 controls the switching part 20 with the switching signal to switch the connection target of the electrode part 10 to the relative dielectric constant calculation unit 40. Next, the controller 50 outputs the voltage acquisition start signal to instruct the second voltage acquisition part 41 to acquire the inter-electrode voltage of the electrode part 10. The second voltage acquisition part 41 acquires the inter-electrode voltage value and outputs the acquired inter-electrode voltage value to the relative dielectric constant calculation part 42. The relative dielectric constant calculation part 42 calculates the relative dielectric constant on the basis of the output inter-electrode voltage value and outputs the relative dielectric constant to the controller 50.

In addition, the controller 50 performs determination of the degradation state, etc. of the oil on the basis of the values of the electrical conductivity and the relative dielectric constant respectively output from the electrical conductivity calculation part 32 and the relative dielectric constant calculation part 42. For example, on the basis of thus-determined degradation state of the oil, the controller 50 displays a warning for a replacement of the oil, for example, on a display part (not shown).

### < Electrical Conductivity Calculation Processing Performed by Fluid State Detection Sensor 1>

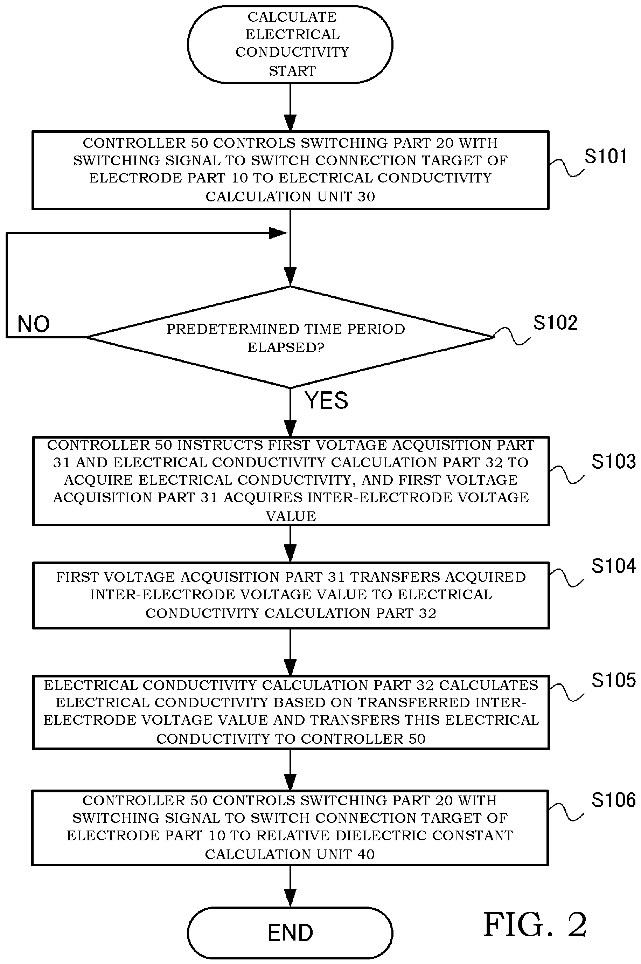

An electrical conductivity calculation processing performed by the fluid state detection sensor 1 according to this embodiment will be described with reference to FIG. 2.

In the electrical conductivity calculation processing performed by the fluid state detection sensor 1, first of all, the controller 50 controls the switching part 20 with the switching signal to switch the connection target of the electrode part 10 from the relative dielectric constant calculation unit 40 to the electrical conductivity calculation unit 30 (Step S101).

Next, the controller 50 determines whether or not a predetermined time period has elapsed after the instruction to switch the switching part 20 (Step S102). In the above description, "the predetermined time period" refers to, as described later, a time period from when the connection target of the electrode part 10 is switched to the electrical conductivity calculation unit 30 to when the influence of the surge voltage accumulated in the inter-electrode voltage is disappeared. When the controller 50 determines that the predetermined time period has not elapsed at the present time point, the controller 50 returns the processing to Step S102 and waits. On the other hand, when the controller 50 determines that the predetermined time period has elapsed at the present time point, the processing proceeds to Step S103. Next, the controller 50 instructs the first voltage acquisition part 31 and the electrical conductivity calculation part 32 to calculate the electrical conductivity, and the first voltage acquisition part 31 acquires the inter-electrode voltage value between the anode 11 and the cathode 12 of the electrode part 10 (Step S103).

The first voltage acquisition part 31 outputs the acquired inter-electrode voltage value to the electrical conductivity calculation part 32 (Step S104). Subsequently, the electrical conductivity calculation part 32 calculates the electrical conductivity on the basis of the output inter-electrode voltage value and outputs the electrical conductivity to the controller 50 (Step S105). Next, the controller 50 controls the switching part 20 with the switching signal, switches the connection target of the electrode part 10 from the electrical conductivity calculation unit 30 to the relative dielectric constant calculation unit 40, and terminates the electrical conductivity calculation processing (Step S106).

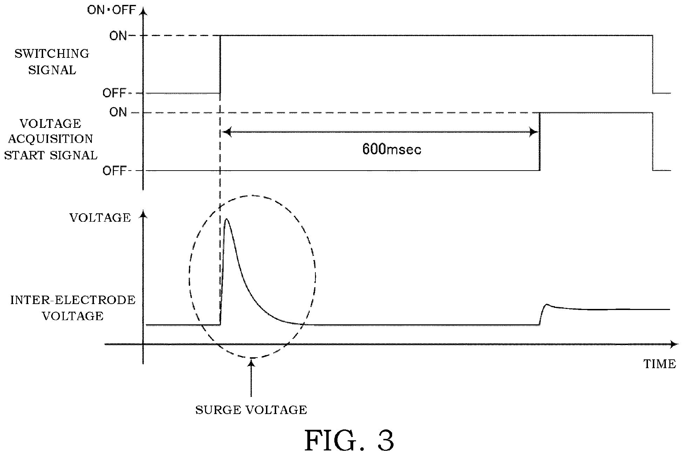

In the above description, FIG. 3 is a diagram illustrating an inter-electrode voltage value acquisition timing and an inter-electrode voltage waveform at the time when the above-described electrical conductivity calculation processing is executed. The switching signal is a signal for controlling the switching part 20 and is a signal for switching the connection target of the electrode part 10. When the electrical conductivity is to be calculated, the controller 50 outputs the switching signal (the ON signal), and the electrode part 10 is connected to the electrical conductivity calculation unit 30.

The voltage acquisition start signal is output from the controller 50 and is a signal for controlling the timing at which the first voltage acquisition part 31 acquires the inter-electrode voltage. When the voltage acquisition start signal (the ON signal) is output, the first voltage acquisition part 31 acquires the inter-electrode voltage value of the electrode part 10. Thus-acquired voltage value is output to the electrical conductivity calculation part 32. The electrical conductivity calculation part 32 calculates the electrical conductivity of the oil that flows between the anode 11 and the cathode 12 of the electrode part 10 on the basis of the voltage value output from the first voltage acquisition part 31. Specifically, the electrical conductivity calculation part 32 obtains the resistance component between the anode 11 and the cathode 12 of the electrode part 10 on the basis of the voltage value output from the first voltage acquisition part 31 and obtains the electrical conductivity of the liquid on the basis of the obtained resistance component.

The inter-electrode voltage shown in FIG. 3 shows the voltage waveform generated between the electrodes of the electrode part 10. When the connection target of the electrode part 10 is switched, the surge voltage may be accumulated in the inter-electrode voltage due to the factors such as the accumulation state of electric charge in the electrodes, the degradation state of the working oil, and so forth, and the inter-electrode voltage may become unstable. As shown in FIG. 3, the surge voltage is generated at the timing when the connection target of the electrode part 10 is switched according to the switching signal, and a large transient voltage variation is caused in the inter-electrode voltage.

However, as shown in FIG. 3, after waited for 600 msec, as the predetermined time period, after the switching of the connection target of the electrode part 10 by the electrical conductivity calculation unit 30 according to the switching signal, the influence of the surge voltage accumulated in the inter-electrode voltage is disappeared. Thus, the first voltage acquisition part 31 can acquire the stabilized inter-electrode voltage value of the electrode part 10 by acquiring the inter-electrode voltage value when, as the predetermined time period, 600 msec has elapsed after the switching part 20 has switched the connection target of the electrode part 10 to the electrical conductivity calculation unit 30.

In the above-described fluid state detection sensor 1, although the predetermined time period (the waiting time) from the switching of the connection target of the electrode part 10 to the acquisition of the inter-electrode voltage value has been described as being 600 msec, the predetermined time period may be changed appropriately. In general, the accumulated level of the surge voltage depends on the resistance value derived from a contaminant component of the oil, and the greater the resistance value is, the higher the surge voltage becomes. Therefore, it is preferable to set the predetermined time period before the acquisition of the inter-electrode voltage value by assuming heavily contaminated oil.

### <Relative Dielectric Constant Calculation Processing Performed by Fluid State Detection Sensor 1>

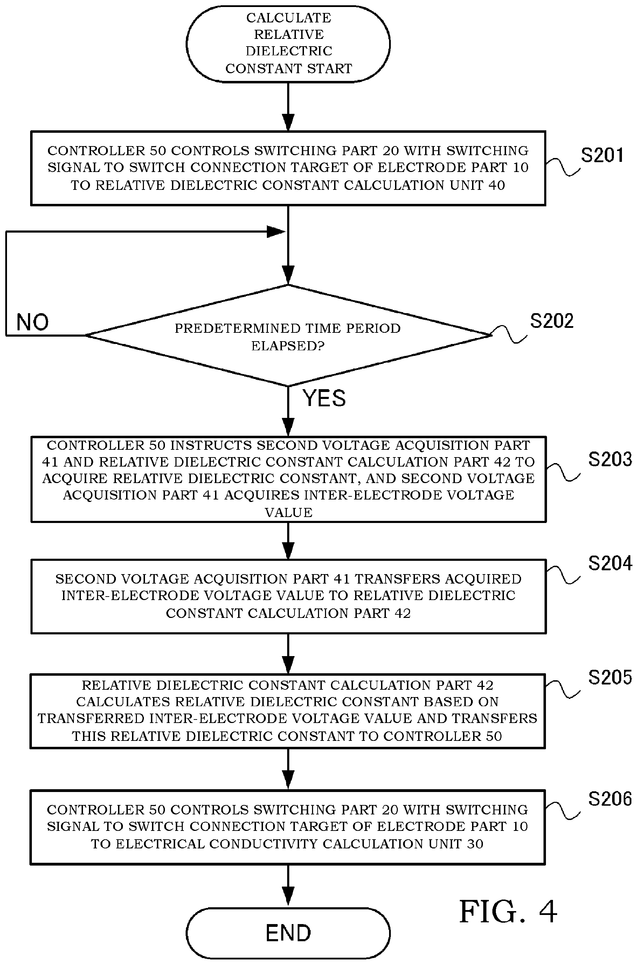

A relative dielectric constant calculation processing performed by the fluid state detection sensor 1 according to this embodiment will be described with reference to FIG. 4.

In the relative dielectric constant calculation processing performed by the fluid state detection sensor 1, first of all, the controller 50 controls the switching part 20 with the switching signal to switch the connection target of the electrode part 10 from the electrical conductivity calculation unit 30 to the relative dielectric constant calculation unit 40 (Step S201).

Next, the controller 50 determines whether or not the predetermined time period has elapsed after the instruction to switch the switching part 20 (Step S202). At this time, when the controller 50 determines that the predetermined time period has not elapsed at the present time point, the controller 50 returns the processing to Step S202 and waits. On the other hand, when the controller 50 determines that the predetermined time period has elapsed at the present time point, the processing proceeds to Step S203. In the above description, the predetermined time period that is set when the relative dielectric constant is to be calculated may be a time period for sufficiently stabilizing the inter-electrode voltage, and it may be different from the predetermined time period that is set for the electrical conductivity calculation processing. Next, the controller 50 instructs the second voltage acquisition part 41 and the relative dielectric constant calculation part 42 to calculate the relative dielectric constant, and the second voltage acquisition part 41 acquires the inter-electrode voltage value between the anode 11 and the cathode 12 of the electrode part 10 (Step S203).

The second voltage acquisition part 41 outputs the acquired inter-electrode voltage value to the relative dielectric constant calculation part 42 (Step S204). Subsequently, the relative dielectric constant calculation part 42 calculates the relative dielectric constant of the oil that flows between the anode 11 and the cathode 12 of the electrode part 10 on the basis of the output inter-electrode voltage value. Specifically, the relative dielectric constant calculation part 42 obtains the resistance component between the anode 11 and the cathode 12 of the electrode part 10 on the basis of the voltage value output from the second voltage acquisition part 41 and obtains the relative dielectric constant of the liquid on the basis of the obtained resistance component. The relative dielectric constant calculation part 42 outputs thus-calculated relative dielectric constant to the controller 50 (Step S205). Next, the controller 50 controls the switching part 20 with the switching signal, switches the connection target of the electrode part 10 from the relative dielectric constant calculation unit 40 to the electrical conductivity calculation unit 30, and terminates the relative dielectric constant calculation processing (Step S206).

According to the above-described embodiment, following operational advantages are afforded.

In the fluid state detection sensor 1, the controller 50 instructs the second voltage acquisition part 41 to acquire the inter-electrode voltage value after waiting for the predetermined time period after the connection target of the electrode part 10 has been switched. In other words, the first voltage acquisition part 31 and the second voltage acquisition part 41 acquire the inter-electrode voltage value after waiting for the predetermined time period after the connection target of the electrode part 10 has been switched. Therefore, it is possible to acquire the inter-electrode voltage value in a state at which the influence of the surge voltage accumulated when the connection target of the electrode part 10 is switched has been disappeared. Therefore, it is possible to suppress deterioration of the measurement accuracies for the electrical conductivity and the relative dielectric constant and to accurately detect the state of the fluid by using the fluid state detection sensor 1.

The predetermined time period that is set for the measurement of the electrical conductivity and the relative dielectric constant performed by the fluid state detection sensor 1 is a time period required to stabilize the inter-electrode voltage value after the connection target of the electrode part 10 has been switched. In other words, the electrical conductivity calculation unit 30 and the relative dielectric constant calculation unit 40 can acquire the inter-electrode voltage value in a state at which the influence of the surge voltage accumulated in the inter-electrode voltage when the connection target of the electrode part 10 is switched has been sufficiently disappeared. Therefore, it is possible to suppress the deterioration of the measurement accuracies for the electrical conductivity and the relative dielectric constant and to accurately detect the state of the fluid by using the fluid state detection sensor 1.

### <Modification>

The processing performed by the above-described fluid state detection sensor 1 may be recorded in a recording medium readable by a computer system, and the program recorded in the recording medium may be read and executed by the controller 50. The fluid state detection sensor 1 of this embodiment can also be realized with such a configuration. In the above description, the computer system includes an operation system and a hardware such as accessory devices.

The configurations, operations, and effects of the embodiment of the present invention configured as described above will be collectively described.

The fluid state detection sensor 1 is provided with: the pair of electrodes 11 and 12 arranged in the flow passage of the fluid, the pair of electrodes 11 and 12 being provided so as to be opposed to each other; the electrical conductivity calculation unit 30 having the first voltage acquisition part 31 configured to acquire the inter-electrode voltage value via the pair of electrodes 11 and 12 and the electrical conductivity calculation part 32 configured to calculate the electrical conductivity of the fluid from voltage value acquired in the first voltage acquisition part 31; the relative dielectric constant calculation unit 40 having the second voltage acquisition part 41 configured to acquire the inter-electrode voltage value via the pair of electrodes 11 and 12 and the relative dielectric constant calculation part 42 configured to calculate the relative dielectric constant of the fluid from the voltage value acquired in the second voltage acquisition part 41; and the switching part 20 configured to switch the connection target of the pair of electrodes 11 and 12 to the electrical conductivity calculation unit 30 or the relative dielectric constant calculation unit 40, wherein the first voltage acquisition part 31 acquires the inter-electrode voltage value after the predetermined time period after the switching part 20 switched the connection target of the pair of electrodes 11 and 12 to the electrical conductivity calculation unit 30, and the second voltage acquisition part 41 acquires the inter-electrode voltage value after the predetermined time period after the switching part 20 switched the connection target of the pair of electrodes 11 and 12 to the relative dielectric constant calculation unit 40.

With this configuration, the first voltage acquisition part 31 and the second voltage acquisition part 41 acquire the inter-electrode voltage value after waiting for the predetermined time period after the connection target of the electrode part 10 has been switched. Therefore, it is possible to acquire the inter-electrode voltage value in a state at which the influence of the surge voltage accumulated when the connection target of the electrode part 10 is switched has been disappeared. Therefore, it is possible to suppress deterioration of the measurement accuracies for the electrical conductivity and the relative dielectric constant and to accurately detect the state of the fluid by using the fluid state detection sensor 1.

In the fluid state detection sensor 1, the predetermined time period is the time period required to stabilize the inter-electrode voltage value.

With this configuration, the electrical conductivity calculation unit 30 and the relative dielectric constant calculation unit 40 can acquire the inter-electrode voltage value in a state at which the influence of the surge voltage accumulated in the inter-electrode voltage when the connection target of the electrode part 10 is switched has been sufficiently disappeared. Therefore, it is possible to suppress the deterioration of the measurement accuracies for the electrical conductivity and the relative dielectric constant and to accurately detect the state of the fluid by using the fluid state detection sensor 1.

Embodiments of the present invention were described above, but the above embodiments are merely examples of applications of the present invention, and the technical scope of the present invention is not limited to the specific constitutions of the above embodiments.

With respect to the above description, the contents of application No. 2020-218839, with a filing date of December 28, 2020 in Japan, are incorporated herein by reference.

## Claims

1. A fluid state detection sensor configured to detect a state of a fluid comprising:
a pair of electrodes arranged in a flow passage of the fluid, the pair of electrodes being provided so as to be opposed to each other;
an electrical conductivity calculation unit having a first voltage acquisition part configured to acquire an inter-electrode voltage value via the pair of electrodes and an electrical conductivity calculation part configured to calculate an electrical conductivity of the fluid from the voltage value acquired in the first voltage acquisition part;
a relative dielectric constant calculation unit having a second voltage acquisition part configured to acquire an inter-electrode voltage value via the pair of electrodes and a relative dielectric constant calculation part configured to calculate a relative dielectric constant of the fluid from the voltage value acquired in the second voltage acquisition part; and
a switching part configured to switch a connection target of the pair of electrodes to the electrical conductivity calculation unit or the relative dielectric constant calculation unit, wherein
the first voltage acquisition part acquires the inter-electrode voltage value after a predetermined time period after the switching part switched the connection target of the pair of electrodes to the electrical conductivity calculation unit, and
the second voltage acquisition part acquires the inter-electrode voltage value after a predetermined time period after the switching part switched the connection target of the pair of electrodes to the relative dielectric constant calculation unit.

2. The fluid state detection sensor according to claim 1, wherein
the predetermined time period is a time period required to stabilize the inter-electrode voltage value.
